# EUROPEAN PATENT SPECIFICATION

(11) **EP 0 495 350 B1**
(45) Date of publication and mention of the grant of the patent: **17.04.1996**
(21) Application number: 92100008.9
(22) Date of filing: 02.01.1992
(51) Int. Cl.: C07C 49/813, C07C 45/46, C07C 45/36, C07C 45/43, C07C 45/00

(54) **Process for the preparation of 4,4'-bis(4-chlorobenzoyl)benzophenone**
Verfahren zur Herstellung von 4,4'-Bis(4-chlorobenzoyl)benzophenon
Procédé de préparation de la 4,4'-bis(4-chlorobenzoyl)benzophénone

(30) Priority: 18.01.1991 JP 16897/91
(43) Date of publication of application: 22.07.1992
(73) Proprietor: KUREHA KAGAKU KOGYO KABUSHIKI KAISHA, Chuo-ku Tokyo 103 (JP)
(72) Inventor: Sunagawa, Kazuhiko, Iwaki-shi, Fukushima-ken (JP); Yoshida, Kazuo, Iwaki-shi, Fukushima-ken (JP); Hoshi, Hajime, Iwaki-shi, Fukushima-ken (JP)
(74) Representative: Kraus, Walter, Dr.

(56) References cited:
- EP-A- 0 004 710
- EP-A- 0 214 565
- FR-A- 2 230 615
- US-A- 4 978 798

## Description

This invention relates to a preparation process for 4,4'-bis(4-chlorobenzoyl)benzophenone, which is useful as a starting material for the production of polymers having excellent heat resistance.

Use of 4,4'-dibenzoylbenzophenone, which does not have any chlorine atom at each end thereof, as a synthesis intermediate for bactericidal compounds is disclosed in U.S. Patent No. 3,808,316. However, the above 4,4'-dibenzoylbenzophenone cannot be used, as it is, as a starting material for the production of polymers. It is also disclosed in the above patent that 4,4'-dibenzoylbezophenone is synthesized by the oxidization of 4,4'-dibenzoyldiphenylmethane obtained by reacting benzoyl chloride with diphenylmethane. The synthesis yield of 4,4'-dibenzoyldiphenylmethane, which is an intermediate, is however as low as about 12%.

EP-A-214 565 relates to a process for the production of bis(fluorobenzoyl) compounds. Said compounds are produced by the reaction of a compound of the general formula p-X-[Ph(R₁)(R₂)]-CO-A-CO-[Ph(R₁)(R₂)]-X-p wherein X represents a bromine or chloride, with an inorganic salt of hydrogen fluoride.

EP-A-004 710 relates to the preparation of 4,4'-difluorobenzophenone which is prepared from 4,4'-diamino-diphenylmethane by diazotization in anhydrous or concentrated aqueous hydrogen fluoride followed by (a) thermal decomposition of the diazonium fluoride thus produced to yield 4,4'-difluoro-diphenylmethane which is subsequently oxidised to yield 4,4'-difluoro-benzophenone or (b) thermal decomposition of the diazonium fluoride, in the presence of nitrous acid or nitrite ions as oxisising agent, to yield 4,4'-difluoro-benzophenone.

US-PS 4 978 789 discloses a method of making a bisphenyl dihalomethane by reacting a trihalomethylbenzene with a halobenzene and a Lewis acid. The bisphenyl dihalomethane can then be reacted with water to form a halobenzophenone. In an optical third step ring chlorines that are present can be replaced with fluorines and, in a final step, any remaining chlorines can be removed to leave a flourobenzophenone. The process is particularly useful in making 4,4'-difluorobenzophenone, which is in turn useful in making polyetheretherketones.

An object of the present invention is therefore to provide a process for the preparation of 4,4'-bis(4-chlorobenzoyl)benzophenone, at a high yield, which is useful as a starting material for heat-resistant polymers.

4,4'-Bis(4-chlorobenzoyl)benzophenone is represented by the following formula: 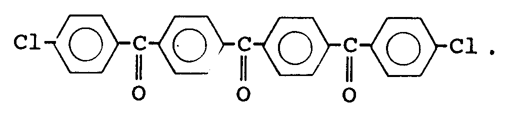
Its detailed physical properties will be described later in Example 1.
For the preparation of 4,4'-bis(4-chlorobenzoyl)-benzophenone according to the present invention, either one of the following processes can be employed. Namely, (1) 4-chlorobenzoyl chloride is reacted with diphenylmethane in the presence of a Lewis acid catalyst, followed by the oxidation of the resultant 4,4'-bis(4-chlorobenzoyl)diphenylmethane with molecular oxygen or (2) 4-chlorobenzotrichloride is reacted with diphenylmethane in the presence of a Lewis acid catalyst, the resultant 4,4'-bis(4-chlorophenyldichloromethyl)diphenylmethane is subjected to hydrolysis and the 4,4'-bis(4-chlorobenzoyl)diphenylmethane thus formed is then oxidized with molecular oxygen.

In the process of the present invention, 4,4'-bis(4-chlorobenzoyl)diphenylmethane, which is the intermediate, is synthesized as described above by the process (1) or (2). Namely, it is obtained directly by reacting 4-chlorobenzoyl chloride with diphenylmethane. As an alternative, it is also synthesized by reacting 4-chlorobenzotrichloride with diphenylmethane and then subjecting the resultant 4,4'-bis(4-chlorophenyldichloromethyl)diphenylmethane to hydrolysis. In either case, 2 moles of 4-chlorobenzoyl chloride or 4-chlorobenzotrichloride are reacted with 1 mole of diphenylmethane. It is preferable to use 4-chlorobenzoyl chloride or 4-chlorobenzotrichloride in a little excess, with 2.2-2.6 moles being particularly preferred.

When 4-chlorobenzoyl chloride is used as a starting material, a reaction solvent is used preferably. 4-Chlorobenzoyl chloride and a desired amount of a Lewis acid catalyst are added, followed by the addition of diphenylmethane under stirring. They are then reacted. The reaction is conducted in a temperature range of from 50°C to 150°C, preferably from 60°C to 110°C, for 1-10 hours, preferably 2-5 hours. The lower the reaction temperature, the less the formation of isomers other than the target product, albeit a longer reaction time is required. Accordingly, it is impractical to conduct the reaction at 50°C or lower. On the other hand, the higher the reaction temperature, the more the formation of isomers. Moreover, temperatures of 150°C and higher result in the formation of other high-boiling products, leading to a reduced yield.

After the completion of the reaction, the Lewis acid catalyst is decomposed by pouring the reaction mixture into ice water or adding water to the reaction mixture. Then, the organic layer is separated and is subjected to distillation. The target compound is then obtained from the residue as a result of removal of the reaction solvent by the distillation. In this case, an extraction solvent such as chloroform be added to the mixed solution after the decomposition of the Lewis acid catalyst, thereby dissolving organic substances in the extraction solvent. The organic layer is then separated from the water layer and then subjected to distillation, whereby both the reaction solvent and the extraction solvent can be removed. The distillation residue is added with toluene, benzene, hexane-ethyl acetate or the like and is subjected to recrystallization, whereby high-purity 4,4'-bis(4-chlorobenzoyl)diphenylmethane can be obtained from the residue.

In the case where 4-chlorobenzotrichloride is employed as a starting material, on the other hand, it is preferable to use a reaction solvent. To the mixture of the reaction solvent, 4-chlorobenzotrichloride and a desired amount of a Lewis acid catalyst, diphenylmethane is added under stirring for reaction. As the reaction velocity is high, it is preferred to conduct the reaction at a temperature as low as possible so that the the formation of isomers other than the target product can be minimized. The reaction is hence conducted in a temperature range of from -20°C to 25°C, preferably 0°C to 10°C. The reaction time is 0.5-10 hours, with 1-5 hours being preferred.

After the completion of the reaction, the Lewis acid catalyst is decomposed gradually by adding water to the reaction mixture and the resultant mixture is heated to a temperature ranging from 80°C to the reflux temperature and then stirred at the same temperature, whereby 4,4'-bis(4-chlorophenyldichloromethyl)diphenylmethane formed by the above reaction is hydrolyzed into 4,4'-bis(4-chlorobenzoyl)diphenylmethane. Separation of 4,4'-bis(4-chlorobenzoyl)diphenylmethane from the reaction mixture may be conducted in a similar manner to the former case in which 4-chlorobenzoyl chloride is employed as a starting material.

Examples of the Lewis acid catalysts used in the above reactions include AlCl₃, FeCl₃, SbCl₃, SnCl₄ and TiCl₄. It is preferable to use the Lewis acid catalyst in an amount equimolar with the starting material, that is, 4-chlorobenzoyl chloride or 4-chlorobenzotrichloride.

Illustrative of the reaction solvents used upon conducting the reactions include 1,1,2,2-tetrachloroethane and tetrachloroethylene. These solvents can be used irrespective of the starting material, in other words, whether 4-chlorobenzoyl chloride or 4-chlorobenzotrichloride is used. When 4-chlorobenzotrichloride requiring at a relatively lower reaction temperature is used as a starting material, carbon disulfide or the like can also be used.

Next, 4,4'-bis(4-chlorobenzoyl)diphenylmethane obtained as described above is subjected to the oxidation reaction, whereby the target compound, 4,4'-bis(4-chlorobenzoyl)benzophenone, is obtained.

The oxidation reaction is conducted in a lower aliphatic monocarboxylic acid as a solvent, said acid having a water content of 40 wt.% or lower, in the presence of a catalyst while using molecular oxygen gas. As the lower aliphatic monocarboxylic acid, that having 1-4 carbon atoms is used. Examples include formic acid, acetic acid, propionic acid and butyric acid, with acetic acid being particularly preferred. As the solvent, a lower aliphatic monocarboxylic acid containing water is preferred because the oxidation yield is improved when the solvent contains some water. A water content of 8-20 wt.% is especially preferred. The existence of water can prevent a carbinol, which is an intermediate of the oxidation reaction, from reacting with such a lower aliphatic monocarboxylic acid as a solvent to form an ester. Water contents higher than 40 wt.% are, however, not preferred because the oxidation reaction is considerably slowed down. The solvent is used in an amount such that the concentration of 4,4'-bis(4-chlorobenzoyl)diphenylmethane becomes 2-30 wt.%, preferably 5-25 wt.%. It is economically disadvantageous to lower the concentration of 4,4'-bis(4-chlorobenzoyl)diphenylmethane below 2 wt.%. On the other hand, if the concentration is higher than 30 wt.%, a reduction in yield occurs. Concentrations outside the above range are therefore not preferred. Concentrations within the range of 5-25 wt.% are especially preferred because the target product can be obtained substantially in a constant yield.

As the catalyst for the oxidation reaction, a cobalt salt catalyst and/or manganese salt catalyst and a bromine catalyst are used. Examples of the cobalt salt and manganese salt catalysts include aliphatic carboxylates and aromatic carboxylates, respectively, such as acetate, propionate, butyrate, benzoate and phthalate. The cobalt salt catalyst and manganese salt catalyst can be used singly or in combination at a desired ratio. They are used in a total amount of 0.0005 gram atom or higher, preferably 0.001-0.2 gram atom in terms of metal elements based on 100 g of the solvent.

As the bromine component of the catalyst, bromine itself and bromine compounds can be used as long as they are soluble in the oxidation reaction solvent and can yield bromide ion. Preferable examples include hydrobromic acid, sodium bromide, potassium bromide, ammonium bromide, cobalt bromide and manganese bromide. The bromine component can be used in an amount of 0.0001 gram atom or higher, preferably 0.001-0.05 gram atom based on 100 g of the solvent.

The higher the partial oxygen pressure in the system, the more rapidly the oxidation reaction proceeds. From the practical viewpoint, the satisfactory partial oxygen pressure is at least 0.1 kg/cm² (absolute), preferably 0.2 kg/cm²-8 kg/cm² (absolute). Air or oxygen gas is used as molecular oxygen gas. When molecular oxygen is used in a form mixed with an inert gas, the reaction proceeds rapidly at an overall partial pressure of 30 kg/cm² G or lower.

The reaction temperature can be 120-250°C, preferably 150-220°C. The reaction time can be about 0.5-10 hours, although it varies depending on the amount of the lower aliphatic carboxylic acid used as a solvent, the amount of the catalyst employed, the reaction temperature, the reaction pressure and the like.

After the completion of the oxidation reaction, the reaction mixture is cooled down to room temperature and crystals thus precipitated are separated, whereby the target product of 4,4'-bis(4-chlorobenzoyl)benzophenone can be obtained. To improve the purity of the product, it is desirable to wash the separated crystals with a solvent capable of dissolving the catalyst therein, such as acetic acid, and then to conduct recrystallization from a solvent such as 1-methyl-2-pyrrolidinone, dimethylacetamide or the like.

4,4'-Bis(4-chlorobenzoyl)benzophenone is useful as a starting material for the production of heat-resistant polymers. It is copolymerizable with other aromatic compounds. For instance, a copolymer available from the copolymerization of 4,4'-bis(4-chlorobenzoyl)benzophenone with an oligomer of phenylenethioether can be a promising candidate for a resin superior to polyphenylenethioether in heat resistance while retaining the excellent properties of polyphenylenethioether such as chemical resistance and crystallinity.

According to the process of the present invention, 4,4'-bis(4-chlorobenzoyl)benzophenone can be prepared in a high yield.

### Examples:

The present invention will hereinafter be described more specifically by examples.

### Example 1

In a 4-necked 500 mℓ flask equipped with a stirrer, a dropping funnel, a thermometer and a reflux condenser, 25.60 g (0.192 mol) of aluminium chloride and 100 mℓ of 1,1,2,2-tetrachloroethane were charged and stirred until aluminium chloride no longer existed in mass. Then, 44.14 g (0.192 mol) of 4-chlorobenzotrichloride was added to the reaction mixture at 5°C. At the same temperature, 13.45 g (0.08 mol) of diphenylmethane was added dropwise to the resultant mixture and reacted for 2 hours under stirring. After the completion of the reaction, 100 mℓ of water was added to the reaction mixture. By raising the temperature of the reaction mixture to 100°C and conducting vigorous stirring for 3 hours, 4,4'-bis(4-chlorophenyldichloromethyl)diphenylmethane formed by the above reaction was hydrolyzed, whereby 4,4'-bis(4-chlorobenzoyl)diphenylmethane was formed.

After the completion of the hydrolysis, the reaction mixture was cooled and added with 200 mℓ of chloroform, whereby organic substances were dissolved. The chloroform layer was separated, washed with water and then subjected to distillation to drive out chloroform and 1,1,2,2-tetrachloroethane. The residue was added with toluene and recrystallized therefrom, whereby 28.0 g of 4,4'-bis(4-chlorobenzoyl)diphenylmethane was obtained as grayish white needle crystals. By high-performance liquid chromatography, the purity and yield were found to be 98.5 % and 77.4%, respectively.

According to NMR, GC-MS, IR and elemental analyses, it was confirmed to be a compound represented by the following structural formula: 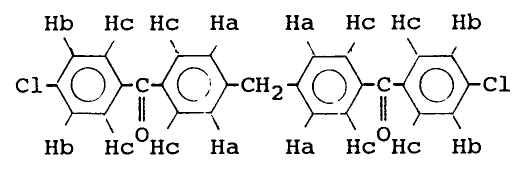
The following are the analytical data of the compound:
¹H NMR (250MHz,CDCl₃)
4.14 ppm (2H,s,-CH₂-)
7.39 ppm (4H,d,Ha)
7.45 ppm (4H,d,Hb)
7.74 ppm (8H,d,Hc)
Molecular weight (Mass) : 444
IR: 1663 cm⁻¹ (C=O)

| Elemental analysis: | | |
|---|---|---|
| | [Calculated] | [Found] |
| C: | 72.8 wt.% | 72.83 wt.% |
| H: | 4.1 wt.% | 4.07 wt.% |
| Cl: | 15.9 wt.% | 15.92 wt.% |

Melting point: 174-175°C
In a titanium-made 200 mℓ autoclave equipped with a stirrer, a reflux condenser and an air feeding tube, 20.00 g of 4,4'-bis(4-chlorobenzoyl)diphenylmethane, 90 g of acetic acid, 10 g of water, 0.5 g of cobalt acetate tetrahydrate, 1.0 g of manganese acetate tetrahydrate and 1.00 g of ammonium bromide were charged, followed by pressurization with nitrogen to 10 kg/cm²G and then heating. After the reaction system was heated to 180°C, air was fed at a rate of 24 ℓ/hour under stirring while maintaining the pressure and temperature at 11 kg/cm²G and at 180°C, respectively. The reaction was continued for 7 hours.

After the completion of the reaction, the reaction mixture was cooled down to room temperature. Crystals thus precipitated were collected by filtration and washed with acetic acid. The crystals thus obtained were recrystallized from 1-methyl-2-pyrrolidinone, whereby 17.53 g of 4,4'-bis(4-chlorobenzoyl)benzophenone was obtained. The purity as measured by a differential scanning calorimeter (manufactured by Mettler Co.) was as high as 99.90%. The yield was 84.9%.

By mass, Ir and elemental analyse, it was confirmed to be a compound represented by the following structural formula: 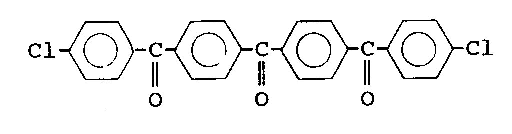
The following are the analytical data of the compound:
Molecular weight (Mass) : 458
IR: 1645 cm⁻¹ (C=O)

| Elemental analysis: | | |
|---|---|---|
| | [Calculated] | [Found] |
| C: | 70.6 wt.% | 70.61 wt.% |
| H: | 3.5 wt.% | 3.51 wt.% |
| Cl: | 15.4 wt.% | 15.44 wt.% |

Melting Point: 310.5-311°C

### Example 2

In a 4-necked 500 mℓ flask equipped with a stirrer, a dropping funnel, a thermometer and a reflux condenser, 25.60 g (0.192 mol) of aluminium chloride, 100 mℓ of 1,1,2,2-tetrachloroethane and 33.60 g (0.192 mol) of 4-chlorobenzoyl chloride were charged and stirred at room temperature until aluminium chloride no longer existed in mass. Then, 13.45 g (0.08 mol) of diphenylmethane was added dropwise to the resultant mixture and reacted at 100°C for 3 hours under stirring. After the completion of the reaction, the reaction mixture was poured into ice water, to which 200 mℓ of chloroform were added to have organic substances dissolved therein. The chloroform layer was separated and subjected to distillation to drive out chloroform and 1,1,2,2-tetrachloroethane. The residue was added with toluene to cause recrystallization, whereby 23.8 g of 4,4'-bis(4-chlorobenzoyl)diphenylmethane was obtained as grayish white needle crystals. By high-performance liquid chromatography, the purity and yield of the crystals were found to be 98.7 % and 66 %, respectively.

In a titanium-made 200 mℓ autoclave equipped with a stirrer, a reflux condenser and an air feeding tube, 20.00 g of 4,4'-bis(4-chlorobenzoyl)diphenylmethane, 85 g of acetic acid, 15 g of water, 0.5 g of cobalt acetate tetrahydrate, 1.0 g of manganese acetate tetrahydrate and 1.00 g of ammonium bromide were charged, followed by pressurization with nitrogen to 10 kg/cm²G and then heating. After the reaction mixture was heated to 180°C, air was fed at a rate of 24 ℓ/hour under stirring while maintaining the pressure and temperature at 11 kg/cm²G and 180°C, respectively.

After the completion of the reaction, the reaction mixture was cooled down to room temperature. Crystals thus precipitated were collected by filtration and washed with acetic acid. The resulting crystals were recrystallized from 1-methyl-2-pyrrolidinone, whereby 17.45 g of 4,4'-bis(4-chlorobenzoyl)benzophenone was obtained. The purity as analyzed by a differential scanning calorimeter (manufactured by Mettler Co.) was as high as 99.90%. The yield was 84.5%.

### Example 3

The synthesis reaction was conducted in a similar manner to Example 2 except that water was not employed as a solvent in the oxidizing reaction. The yield of the oxidizing reaction was 75.3 %.

### Example 4

In a 4-necked 300 mℓ flask equipped with a stirrer, a dropping funnel and a thermometer, 12.80 g (0.096 mol) of aluminium chloride and 100 mℓ of carbon disulfide were charged and cooled down to 5°C. Thereafter, the mixture was added with 22.07 g (0.096 mol) of 4-chlorobenzotrichloride, followed by stirring for 30 minutes. At the same temperature, 6.72 g (0.04 mol) of diphenylmethane were added dropwise to the reaction mixture. The reaction was thereafter allowed to proceed for 2 hours.

After the completion of the reaction, the reaction mixture was poured into ice water, to which 100 mℓ of chloroform were added to have organic substances dissolved therein. The chloroform layer was separated and subjected to distillation to drive out chloroform and carbon disulfide. The residue was added with 10 mℓ of diethylene glycol dimethyl ether and 100 m ℓ of 1N hydrochloric acid. The resulting mixture was refluxed for 3 hours under stirring, whereby hydrolysis was conducted. The hydrolysis product was extracted with chloroform. The extract was washed with water and, then, chloroform was distilled off. The residue thus obtained was recrystallized from toluene, whereby 13.0 g of 4,4'-bis(4-chlorobenzoyl)diphenylmethane were obtained. By high-performance liquid chromatography, the purity and yield were found to be 99.2% and 72.4%.

The resulting 4,4'-bis(4-chlorobenzoyl)diphenylmethane was oxidized in a similar manner to Example 1, whereby 4,4'-bis(4-chlorobenzoyl)benzophenone (yield: 84.9% purity: 99.90%) was obtained, respectively.

## Claims

1. A process for the preparation of 4,4'-bis(4-chlorobenzoyl)benzophenone, which comprises reacting 4-chlorobenzoyl chloride with diphenylmethane in the presence of a Lewis acid catalyst and oxidizing the resulting 4,4'-bis(4-chlorobenzoyl)diphenylmethane with molecular oxygen.

2. The process of claim 1, wherein the reaction with diphenylmethane is conducted in 1,1,2,2-tetrachloroethane.

3. The process of claim 1, wherein 4,4'-bis(4-chlorobenzoyl)diphenylmethane is oxidized with molecular oxygen in the presence of a cobalt salt catalyst and/or manganese salt catalyst and a bromine catalyst in acetic acid as a solvent, said acetic acid having a water content of 40 wt.% or lower.

4. A process for the preparation of 4,4'-bis(4-chlorobenzoyl)benzophenone, which comprises reacting 4-chlorobenzotrichloride with diphenylmethane in the presence of a Lewis acid catalyst, subjecting the resulting 4,4'-bis(4-chlorophenyldichloromethyl)diphenylmethane to hydrolysis and then oxidizing 4,4'-bis(4-chlorobenzoyl)diphenylmethane thus formed with molecular oxygen.

5. The process of claim 4, wherein the reaction with diphenylmethane was conducted in 1,1,2,2-tetrachloroethane.

6. The process of claim 4, wherein 4,4'-bis(4-chlorobenzoyl)diphenylmethane is oxidized with molecular oxygen in the presence of a cobalt salt catalyst and/or manganese salt catalyst and a bromine catalyst in acetic acid as a solvent, said acetic acid having a water content of 40 wt.% or less.

## Patentansprüche

1. Verfahren zur Herstellung von 4,4'-Bis(4-chlorbenzoyl)benzophenon, dadurch **gekennzeichnet,** daß 4-Chlorbenzoylchlorid mit Diphenylmethan in Anwesenheit eines Lewis-Säure-Katalysators umgesetzt wird und das entstehende 4,4'-Bis(4-chlorbenzoyl)diphenylmethan mit molekularem Sauerstoff oxidiert wird.

2. Verfahren nach Anspruch 1, dadurch **gekennzeichnet,** daß die Reaktion mit Diphenylmethan in 1,1,2,2-Tetrachlorethan durchgeführt wird.

3. Verfahren nach Anspruch 1, dadurch **gekennzeichnet,** daß 4,4'-Bis(4-chlorbenzoyl)diphenylmethan mit molekularem Sauerstoff in Anwesenheit eines Kobaltsalz-Katalysators und/oder Mangansalz-Katalysators und eines Brom-Katalysators in Essigsäure als Lösungsmittel oxidiert wird, wobei die Essigsäure einen Wassergehalt von 40 Gew.-% oder darunter besitzt.

4. Verfahren zur Herstellung von 4,4'-Bis(4-chlorbenzoyl)benzophenon, dadurch **gekennzeichnet,** daß 4-Chlorbenzotrichlorid mit Diphenylmethan in Anwesenheit eines Lewis-Säure-Katalysators umgesetzt wird, das entstehende 4,4'-Bis(4-chlorphenyldichlormethyl)diphenylmethan der Hydrolyse unterworfen wird und dann das so gebildete 4,4'-Bis(4-chlorbenzoyl)diphenylmethan mit molekularem Sauerstoff oxidiert wird.

5. Verfahren nach Anspruch 4, dadurch **gekennzeichnet,** daß die Reaktion mit Diphenylmethan in 1,1,2,2-Tetrachlorethan durchgeführt wird.

6. Verfahren nach Anspruch 4, dadurch **gekennzeichnet,** daß 4,4'-Bis(4-chlorbenzoyl)diphenylmethan mit molekularem Sauerstoff in Anwesenheit eines Kobaltsalz-Katalysators und/oder eines Mangansalz-Katalysators und eines Brom-Katalysators in Essigsäure als Lösungsmittel oxidiert wird, wobei die Essigsäure einen Wassergehalt von 40 Gew.-% oder weniger besitzt.

## Revendications

1. Procédé pour la préparation de 4,4'-bis-(4-chlorobenzoyl)-benzophénone, qui comprend la réaction du chlorure de 4-chlorobenzoyle avec du diphénylméthane en présence d'un catalyseur de type acide de Lewis et l'oxydation du 4,4'-bis-(4-chlorobenzoyl)-diphénylméthane résultant avec de l'oxygène moléculaire.

2. Procédé suivant la revendication 1, dans lequel la réaction avec du diphénylméthane est conduite dans du 1,1,2,2-tétrachloroéthane.

3. Procédé suivant la revendication 1, dans lequel le 4,4'-bis-(4-chlorobenzoyl)-diphénylméthane est oxydé avec de l'oxygène moléculaire en présence d'un catalyseur au sel de cobalt et/ou d'un catalyseur au sel de manganèse et d'un catalyseur au brome dans de l'acide acétique utilisé comme solvant, cet acide acétique ayant une teneur en eau de 40% en poids ou moins.

4. Procédé pour la préparation de 4,4'-bis-(4-chlorobenzoyl)-benzophénone, qui comprend la réaction du 4-chlorobenzotrichlorure avec du diphénylméthane en présence d'un catalyseur de type acide de Lewis, la soumission du 4,4'-bis-(4-chlorophényldichlorométhyl)-diphénylméthane résultant à une hydrolyse et ensuite l'oxydation du 4,4'-bis-(4-chlorobenzoyl)-diphénylméthane résultant avec de l'oxygène moléculaire.

5. Procédé suivant la revendication 4, dans lequel la réaction avec du diphénylméthane est conduite dans du 1,1,2,2-tétrachloroéthane.

6. Procédé suivant la revendication 4, dans lequel le 4,4'-bis-(4-chlorobenzoyl)-diphénylméthane est oxydé avec de l'oxygène moléculaire en présence d'un catalyseur au sel de cobalt et/ou d'un catalyseur au sel de manganèse et d'un catalyseur au brome dans de l'acide acétique utilisé comme solvant, cet acide acétique ayant une teneur en eau de 40% en poids ou moins.
